# FASCICULE DE BREVET EUROPEEN

(11) **EP 0 818 451 B1**
(45) Date de publication et mention de la délivrance du brevet: **13.06.2001**
(21) Numéro de dépôt: 97401561.2
(22) Date de dépôt: 02.07.1997
(51) Int. Cl.: C07D 307/83

(54) **Procédé de préparation de l'énollactone de l'acide 2-oxocyclohexylidène acétique et application à la préparation de la 2-coumaranone**
Verfahren zur Herstellung vom Enollakton der 2-Oxycyclohexyliden-Essigsäure und Verwendung zur Herstellung vom 2-Coumaranon
Process for the preparation of the enollactone of the 2-oxocyclohexylidene acetic acid and use for the preparation of the 2-coumaranone

(30) Priorité: 09.07.1996 FR 9608528
(43) Date de publication de la demande: 14.01.1998
(73) Titulaire: Clariant (France) S.A., 92800 Puteaux (FR)
(72) Inventeur: Carmona, Nathalie, 60280 Venette (FR); Carmona, Laurent, 60280 Venette (FR); Perrard, Alain, 69110 Sainte Foy Les Lyon (FR); Vallejos, Jean-Claude, 13005 Marseille (FR)
(74) Mandataire: Rinuy, Santarelli

(56) Documents cités:
- DE-B- 1 184 774
- DE-C- 584 372
- FR-A- 2 721 609
- GB-A- 1 337 507
- US-A- 3 862 133

## Description

La présente invention a pour objet un procédé de préparation de l'énol-lactone de l'acide 2-oxocyclohexylidèneacétique et son application à la préparation de la 2-coumaranone, désignée également 3H-benzofuranone-2.

La 2-coumaranone est un produit connu, largement décrit dans la littérature (cf. Beil. 17, 309 ; I, 159 ; II, 331). C'est la lactone de l'acide orthohydroxyphénylacétique et elle est utilisée comme matière première en synthèse organique pour accéder à divers produits destinés à l'agriculture ou présentant des effets physiologiques. De ce fait, on recherche en permanence des procédés permettant de l'obtenir rapidement et à bon marché à partir de produits commerciaux peu onéreux. Or, la demanderesse a découvert avec étonnement un procédé de préparation industriel de la 2-coumaranone à partir d'acide glyoxylique et de cyclohexanone passant par la préparation de l'énol-lactone de l'acide 2-oxocyclohexylidèneacétique.

Jusqu'à maintenant, les méthodes de synthèse décrites dans la littérature n'ont pas permis la synthèse industrielle de la 2-coumaranone. Parmi les synthèses trouvées, on peut citer celle partant de la lactone de l'acide cis 2-hydroxy-2-acétoxy cyclohexylidèneacétique (A. Mondon et al, Ber. 1963, 96, 826-838), celle partant de l'ortho-cresol (H.E. Holmquist J.Org. Chem. (1969), 34 (12), 4164-5), celle partant du trans-2-3-dichloro 2,3-dihydrobenzofurane (E. Baciocchi et al, J. Org. Chem (1979), 44(1), 32-4), celle partant de l'acide phénylacétique (I. Fukagawa, J. Org.Chem. (1982), 47 (12), 2491-3), celle partant du 9,9-dichloro -7-oxabicyclo [4,3,0] nonane-8-one (N. Taichi et al Nippon Kagaku Kaishi (1984), (8), 1287-92).

Assez récemment, J.C. Vallejos et al (FR 2.686.880) ont décrit une méthode de préparation de la 5-chloro-3H-benzofuranone-2 à partir d'acide glyoxylique et de p. chlorophénol en présence d'acide phosphinique et de quantités catalytiques d'iode ou d'acide iodhydrique. Ils obtiennent un mélange de 5-chloro-3-H-benzofuranone-2 et de p.chlorophénol qui doit être distillé.

Plus récemment, J.C. Vallejos et al. (FR 2.721.609) ont décrit une méthode de préparation de la 2-coumaranone à partir de la déshydrogénation catalytique en phase vapeur du brut réactionnel obtenu à partir de la condensation de l'acide glyoxylique avec la cyclohexanone en milieu acide acétique pur.

Ce brut réactionnel se compose d'un mélange d'acide (oxo-2-cyclohexylidène) acétique trans (appelé par la suite composé trans), de la lactone de l'acide (dihydroxy-2,2 cyclohexylidène) acétique cis (appelée par la suite composé cis) et de l'énol-lactone de l'acide 2-oxocyclohexylidèneacétique (appelée par la suite composé énol-lactone). La déshydrogénation catalytique est effectuée sur catalyseur à base de palladium déposé sur charbon ou alumine.

Les trois inconvénients majeurs du procédé décrit sont la faible productivité de la déshydrogénation, la décomposition des produits du brut réactionnel notamment du produit trans introduisant une désactivation du catalyseur, des réactions de décarboxylation déshydrogénante des composés du brut réactionnel conduisant à la formation d'orthocrésol comme sous produit en début de réaction.

Il serait donc souhaitable de trouver un procédé permettant de pallier ces inconvénients.

La présente invention a pour objet un procédé de préparation de l'énol-lactone de l'acide 2-oxocyclohexylidèneacétique caractérisé par le fait
i) que l'on fait réagir l'acide glyoxylique sur de la cyclohexanone en présence d'un acide halogéné afin d'obtenir un brut de réaction B1, puis
ii) que l'on fait réagir ce brut de réaction B1 après solubilisation dans un solvant organique en présence d'un acide fort soluble dans ledit solvant organique ou de résines fortement acides afin d'obtenir l'énol-lactone de l'acide 2-oxocyclohexylidèneacétique attendu que l'on isole si désiré.

Dans la première étape, on fait réagir l'acide glyoxylique avec de la cyclohexanone en présence d'un acide halogéné, de préférence à une température supérieure à 50°C, en présence ou non d'un solvant pour obtenir un brut réactionnel B1. Celui-ci contient principalement du composé cis, un peu du composé énol-lactone et très peu du composé trans.

Dans la deuxième étape, on déshydrate le brut réactionnel B1, pour obtenir le composé énol-lactone pur si désiré après isolation. Cette déshydratation est réalisée en présence d'un acide fort soluble dans le solvant organique ou de résines fortement acides, de préférence à une température supérieure à 50°C, notamment en présence d'un solvant benzénique.

Le composé de type énol-lactone a été décrit à plusieurs reprises, Yu Wang, Hua Hsueh Hsueh Pao 26, N°2, 84-99 (1960) - M.N. Kolosov Zh. Obshch.Khim. 32, 2893-905 (1962) - R.W. Saalfrank, Chem. Ber. 1983, 116(4), 1463-7 - T. Nakano, J.C.S., Chem. Commun., 1981, 815-816 - Yu. A. Arbuzov, Zh. Obshch. Khim. 32, 3676-81 (1962) - L. Baiocchi, Synthesis 1979, 434-6 - A. Mondon, Ber. 1963, 96, 826-839 - M.M. Shemyakin, Doklady Akad. Nauk. SSSSR, 128, 744-7 (1959) - G. Klotmann, DE 1.955.375 (1969), mais aucune des références n'indique une synthèse produisant le produit pur avec un bon rendement et à un prix raisonnable, transposable industriellement.

Dans des conditions préférentielles de mise en oeuvre de l'invention, le procédé ci-dessus décrit est réalisé de la manière suivante :

Dans la première étape on fait réagir l'acide glyoxylique avec de la cyclohexanone :
- à une température supérieure à 50°C, de préférence supérieure à 80°C, tout particulièrement entre 100°C et 110°C.
- en présence d'un acide halogéné qui est l'acide chlorhydrique à raison de 1 à 100 %, de préférence de 10 à 25% molaire par rapport à l'acide glyoxylique,
- en présence ou non d'un solvant organique ou non tel que le toluène, l'eau, l'acide acétique,...
- avec un rapport molaire cyclohexanone/acide glyoxylique supérieur ou égal à 1, de préférence compris entre 1 et 2,
et l'on obtient un brut réactionnel B1 contenant principalement du composé cis, un peu du composé énol-lactone et très peu de composé trans;

Dans la deuxième étape, on déshydrate le brut réactionnel B1:
- à une température supérieure à 50°C, tout particulièrement entre 80°C et 160°C
- en présence d'un acide fort soluble en milieu organique (acide p-toluène sulfonique, acide méthanesulfonique, ...) ou de résines fortement acides (type Amberlist ® de Rohm et Haas par exemple)
- de préférence en présence d'un solvant benzénique.

Le procédé ci-dessus présente un intérêt tout particulier dans la préparation de la 2-coumaranone, désignée également 3H-benzofuranone-2. En effet, l'énol lactone peut être facilement transformée par déshydrogénation en 2-coumaranone.

La présente invention a donc encore pour objet un procédé ci-dessus, caractérisé en ce qu'on isole l'énol lactone et qu'en outre on procède à la déshydrogénation catalytique en phase vapeur du composé énol-lactone en présence d'un catalyseur de déshydrogénation.

Par l'expression "catalyseur de déshydrogénation", on désigne un catalyseur connu pour l'aromatisation des cycles à 6 chaînons comme ceux cités dans Advanced Organic Chemistry, Jerry March, 3ème édition, pages 1052-1054, J. Willey, Interscience, New-York, 1985, ainsi que dans Houben-Weyl, Phenole, Tome 2, pages 701-716, Georg Thieme - Stuttgart, 1976.

Préférentiellement, le catalyseur de déshydrogénation est choisi dans le groupe constitué par le palladium et le platine.

Dans des conditions préférentielles de mise en oeuvre, le composé énol lactone est déshydrogéné:
- par vaporisation à une température supérieure ou égale à 150°C, tout particulièrement entre 200°C et 300°C,
- entraîné sur le catalyseur à l'aide d'un gaz vecteur constitué de préférence par du diazote,
- en présence d'un catalyseur à base de platine ou de palladium déposé sur un support solide inerte tel que l'alumine α, l'alumine y, la silice, le carbure de silicium, le charbon, présentant une surface spécifique supérieure à 1m² par gramme, à une concentration supérieure ou égale à 0,5 %, tout particulièrement comprise entre 0,5 et 5%.

Le mélange gazeux analysé par chromatographie après condensation dans des pièges contenant de l'acétonitrile peut être distillé pour conduire à la 2-coumaranone isolée.

L'originalité de l'objet de la présente demande de brevet par rapport au brevet FR 2.721.609 de J.C. Vallejos partant des mêmes produits de départ, cyclohexanone et acide glyoxylique, réside dans le fait que l'on a pu produire le composé énol-lactone isolé avec un remarquable rendement. Ce produit est stable, ne se décompose pas et n'empoisonne pas le catalyseur. Il est liquide et ne nécessite pas l'utilisation de solvant. Il est facilement vaporisable et la réaction peut être effectuée avec un faible débit gazeux. On obtient une meilleure productivité en 2-coumaranone tout en ayant une bonne stabilité du catalyseur dans le temps (pas de phénomène d'empoisonnement de celui-ci) par déshydrogénation catalytique en phase vapeur du composé énol-lactone isolé.

Les exemples suivants illustrent la présente invention sans toutefois la limiter.

### EXEMPLE 1 :

### Préparation de l'énol-lactone de l'acide 2-oxocyclohexylidèneacétique.

Dans un ballon de 1 litre, sont introduits :
- 148 g d'acide glyoxylique à 50 % (1 mole)
- 147 g de cyclohexanone (1,5 mole)
- 74 g d'eau
- 10 g d'HCl en solution à 37 % dans l'eau

La solution est ensuite portée au reflux pendant 2 heures puis concentrée sous pression réduite de 0, 027 bar (20 mm Hg) pour éliminer l'eau, l'acide chlorhydrique et la cyclohexanone en excès.

On obtient un mélange constitué de 82 % de composé cis, 12 % de composé de type énol-lactone, 1,6 % de composé trans, cette composition ayant été déterminée par HPLC .

Le mélange est alors solubilisé dans 250 ml de xylène en présence de 10 g d'acide p-toluène sulfonique (APTS).

On élimine ensuite l'eau présente par distillation azéotropique avec le xylène.

Après neutralisation, le mélange brut est alors distillé sous pression réduite de 0,005 bar (4 mm Hg) et on obtient 118 g (0,87 mole) de composé de type énol-lactone (point d'ébullition 120°C sous 0,005 bar (4 mm Hg)), soit un rendement de 87 % par rapport à l'acide glyoxylique initial.

### EXEMPLE 2 :

### Variante de préparation de l'énol-lactone de l'acide 2-oxocyclohexylidèneacétique

Dans un ballon de 1 litre sont introduits :
- 148 g d'acide glyoxylique à 50 % (1 mole)
- 196 g de cyclohexanone (2 moles)
- **74 g d'eau**
- 25 g d'HCl en solution à 37 % dans l'eau

La solution est ensuite portée au reflux pendant 2 heures, puis concentrée sous pression réduite de 0,027 bar (20 mm de Hg) pour éliminer l'eau, l'acide chlorhydrique et le cyclohexanone en excès.

On obtient un mélange constitué de 77 % de composé cis, 20 % de composé type énol-lactone, 2 % de composé trans, cette composition ayant été déterminée par HPLC.

On solubilise alors le mélange dans 250 ml de toluène en présence de 20 g d'une résine sulfonique Amberlyst ® 15. On élimine ensuite l'eau par distillation azéotropique avec le toluène.

Après filtration, le mélange brut est alors distillé sous pression réduite de 0,005 bar (4 mm de Hg) et on obtient 122 g (0,90 mole) de composé de type énol-lactone (point d'ébullition 120°C sous 0,005 bar (4 mm de Hg)), soit un rendement de 90 % par rapport à l'acide glyoxylique initial.

### EXEMPLE 3 :

### Obtention de la 2-coumaranone à partir de l'énol-lactone de l'acide 2-oxo-cyclohexylidène isolée.

Dans un vaporisateur maintenu à une température de 220°C on vaporise une solution S1 exclusivement constituée d'énol-lactone de l'acide 2-oxocyclohexylidène acétique avec un débit de 12 ml/h et les vapeurs sont entraînées par un courant d'azote avec un débit de 200 l/h vers le réacteur maintenu à la température de 250°C.

Le réacteur est un cylindre de diamètre intérieur de 26 mm et de longueur 200 mm, vertical, bouché à son extrémité inférieure par un verre fritté, de volume utile de 80 ml. Il est rempli par un catalyseur de type Pd/Al₂O₃ (30 g de catalyseur soit 150 mg de Pd). A la sortie du réacteur, les gaz chauds sont refroidis et condensés par passage dans des pièges contenant de l'acétonitrile, puis analysés par chromatographie en phase liquide à haute performance (CLHP) et par chromatographie en phase gazeuse (CPG).

Le rendement et la productivité en 2-coumaranone sont définis respectivement comme :
- R (coum) = (nbre de moles de 2-coumaranone) / (nbre de moles de réactifs) exprimé en %
- Productivité = (masse de 2-coumaranone) / (temps de la réaction) / (masse de palladium engagée) exprimée en g/h/g de Pd.

Les résultats obtenus sont résumés dans le tableau I

**Tableau 1:**

| ***déshydrogénation catalytique de l'énol-lactone*** | | |
|---|---|---|
| **t(h)** | **R(coum)** | **Productivité (g/h/g de Pd)** |
| 1 | 71 | 54 |
| 2 | 68 | 52 |
| 4 | 69 | 53 |
| 6 | 69 | 52 |
| 8 | 66 | 51 |

L'utilisation de l'énol-lactone pure comme substrat permet donc d'obtenir :
- une productivité élevée supérieure à 50 g/h/g de Pd
- une bonne stabilité du catalyseur utilisé : le produit de départ étant stable, il n'y a pas d'empoisonnement du catalyseur.
- une réaction en l'absence de solvant d'où un piégeage facile de l'aérosol
- des conditions expérimentales favorables grâce à une vaporisation facile du produit et à la possibilité d'utiliser de faibles débits gazeux.

### EXEMPLE 4

### Obtention de la 2-coumaranone à partir d'un mélange issu de la condensation de la cyclohexanone et de l'acide glyoxylique dans des conditions analogues à celles décrites dans le brevet FR 2.721.609.

Dans des conditions identiques à celles de l'exemple 3 on utilise une solution S2 contenant
- 18,5 % de cis
- 16,2 % de trans
- 0,4 % d'énol-lactone
- 64,9 % d'acide acétique.

Les résultats obtenus sont résumés dans le tableau Il.

**Tableau 2:**

| ***déshydrogénation catalytique d'α-carboxyméthylidène cyclohexanones*** | | |
|---|---|---|
| **t(h)** | **R(coum)** | **Productivité (g/h/g de Pd)** |
| 1 | 34 | 9 |
| 2 | 4,3 | 1,2 |

On voit que le rendement et la productivité en 2-coumaranone sont très inférieurs déjà au bout de 2 heures par rapport aux résultats obtenus avec le présent procédé décrit.

A l'opposé, on se rend compte que même après 8 heures de réaction avec le présent procédé, le rendement et la productivité en 2-coumaranone sont toujours très bons (voir tableau 1).

## Revendications

1. Procédé de préparation de l'énol-lactone de l'acide 2-oxocyclohexylidèneacétique caractérisé par le fait
i) que l'on fait réagir l'acide glyoxylique sur de la cyclohexanone en présence d'un acide halogéné afin d'obtenir un brut de réaction B1, puis
ii) que l'on fait réagir ce brut de réaction B1 après solubilisation dans un solvant organique en présence d'un acide fort soluble dans ledit solvant organique ou de résines fortement acides afin d'obtenir l'énol-lactone de l'acide 2-oxocyclohexylidèneacétique que l'on isole si désiré.

2. Procédé selon la revendication 1, caractérisé par le fait que l'acide halogéné de l'étape i) est l'acide chlorhydrique, à raison de 1 à 100 % molaire par rapport à l'acide glyoxylique.

3. Procédé selon la revendication 1 ou 2, caractérisé par le fait que l'étape i) est effectuée à une température supérieure à 50°C.

4. Procédé selon l'une des revendications 1 à 3, caractérisé par le fait que le solvant organique de solubilisation du brut B1 dans l'étape ii) est un solvant benzénique.

5. Procédé selon l'une des revendications 1 à 4, caractérisé par le fait que l'on utilise un acide fort qui est l'acide p.toluène sulfonique ou l'acide méthane sulfonique.

6. Procédé selon l'une des revendications 1 à 4, caractérisé par le fait que l'on utilise une résine fortement acide qui est une résine sulfonique de type Amberlist ®.

7. Procédé selon l'une des revendications 1 à 6, caractérisé en ce qu'on isole l'énol lactone et qu'en outre on procède à la déshydrogénation catalytique en phase vapeur du composé énol-lactone en présence d'un catalyseur de déshydrogénation pour obtenir la 2-coumaranone.

8. Procédé selon la revendication 7, caractérisé par le fait que la réaction en phase vapeur est réalisée à une température supérieure ou égale à 150°C.

9. Procédé selon la revendication 7 ou 8, caractérisé par le fait que le catalyseur de déshydrogénation est choisi dans le groupe constitué par le palladium et le platine.

10. Procédé selon l'une des revendications 7 à 9 caractérisé par le fait que le catalyseur de déshydrogénation est déposé sur un support solide inerte présentant une surface spécifique supérieure à 1m²/g.

## Claims

1. Method of preparing 2-oxocyclohexylideneacetic acid enol lactone, characterised by the fact that
i) glyoxylic acid is caused to react on cyclohexanone in the presence of a halogenated acid in order to obtain a raw reaction product B1, and then
ii) this raw reaction product B1 is caused to react, after solubilisation in an organic solvent in the presence of a strong acid soluble in the said organic solvent or highly acid resins in order to obtain 2-oxocyclohexylideneacetic acid enol lactone, which is isolated if required.

2. Method according to Claim 1, characterised by the fact that the halogenated acid of step i) is hydrochloric acid, at the rate of 1 to 100% molar with respect to the glyoxylic acid.

3. Method according to Claim 1 or 2, characterised by the fact that step i) is performed at a temperature above 50°C.

4. Method according to one of Claims 1 to 3, characterised by the fact that the organic solvent for solubilising the raw product B1 in step ii) is a benzene solvent.

5. Method according to one of Claims 1 to 14, characterised by the fact that a strong acid is used which is p-toluenesulphonic acid or methanesulphonic acid.

6. Method according to one of Claims 1 to 4, characterised by the fact that a highly acidic resin is used which is a sulphonic resin of the Amberlist® type.

7. Method according to one of Claims 1 to 6, characterised in that the enol lactone is isolated and in addition the enol lactone compound is subjected to vapour phase catalytic dehydrogenation in the presence of a dehydrogenation catalyst in order to obtain 2-coumaranone.

8. Method according to Claim 7, characterised by the fact that the vapour phase reaction is carried out at a temperature greater than or equal to 150°C.

9. Method according to Claim 7 or 8, characterised by the fact that the dehydrogenation catalyst is chosen from the group consisting of palladium and platinum.

10. Method according to one of Claims 7 to 9, characterised by the fact that the dehydrogenation catalyst is deposited on an inert solid support having a specific surface area greater than 1 m²/g.

## Patentansprüche

1. Verfahren zur Herstellung des Enol-Lactons von 2-Oxocyclohexylidenessigsäure dadurch gekennzeichnet,
i) daß man Glyoxylsäure mit Cyclohexanon in Gegenwart einer Halogensäure umsetzt, um ein Reaktionsrohprodukt B1 zu erhalten und dann
ii) daß man dieses Reaktionsrohprodukt B1 nach Lösen in einem organischen Lösungsmittel in Gegenwart einer in dem organischen Lösungsmittel löslichen starken Säure oder von stark sauren Harzen umsetzt, um das Enol-Lacton der 2-Oxocyclohexylidenessigsäure zu erhalten, das man, wenn gewünscht, isoliert.

2. Verfahren nach Anspruch 1, dadurch gekennzeichnet, daß die Halogensäure in Schritt i) Salzsäure darstellt mit einem Molverhältnis von 1 bis 100 %, bezogen auf die Glyoxylsäure.

3. Verfahren nach Anspruch 1 oder 2, dadurch gekennzeichnet, daß man Schritt i) bei einer Temperatur über 50°C durchführt.

4. Verfahren nach einem der Ansprüche 1 bis 3, dadurch gekennzeichnet, daß das organische Lösungsmittel zur Lösung des Rohprodukts B1 im Schritt ii) ein benzolisches Lösungsmittel darstellt.

5. Verfahren nach einem der Ansprüche 1 bis 4, dadurch gekennzeichnet, daß man eine starke Säure einsetzt, die p-Toluolsulfonsäure oder Methansulfonsäure darstellt.

6. Verfahren nach einem der Ansprüche 1 bis 4. dadurch gekennzeichnet, daß man ein stark saures Harz einsetzt, das ein Sulfonharz vom Amberlist® -Typ darstellt.

7. Verfahren nach einem der Ansprüche 1 bis 6. dadurch gekennzeichnet, daß man das Enol-Lacton isoliert und daß man außerdem mit der Enol-Lacton-Verbindung eine katalytische Dehydrierung in der Dampfphase in Gegenwart eines Dehydrierungskatalysators durchführt, um 2-Cumaranon zu erhalten.

8. Verfahren nach Anspruch 7, dadurch gekennzeichnet, daß man die Umsetzung in der Dampfphase bei einer Temperatur über oder gleich 150°C durchführt.

9. Verfahren nach Anspruch 7 oder 8, dadurch gekennzeichnet daß der Dehydrierungskatalysator ausgewählt wird aus der Gruppe, bestehend aus Palladium und Platin.

10. Verfahren nach einem der Ansprüche 7 bis 9, dadurch gekennzeichnet, daß der Dehydrierungskatalysator auf einem festen, inerten Träger abgeschieden ist, der eine spezifische Oberfläche von mehr als 1 m²/ g aufweist.
